# EUROPEAN PATENT SPECIFICATION

(11) **EP 1 973 467 B1**
(45) Date of publication and mention of the grant of the patent: **16.10.2013**
(21) Application number: 07756376.5
(22) Date of filing: 18.01.2007
(51) Int. Cl.: A61B 5/00, G01N 21/47, G01B 9/02

(54) **Systems and process for providing speckle reduction using a wave front modulation for optical coherence tomography**
Systeme und Verfahren zur Bereitstellung von Speckle-Reduktion über eine Wellenfront -Modulation zur optischen Kohärenzentomographie
Systèmes et procédé pour la réduction de speckle au moyens d'une modulation de front d'onde pour la tomographie par cohérence optique

(30) Priority: 20.01.2006 US 760592 P
(43) Date of publication of application: 01.10.2008
(73) Proprietor: The General Hospital Corporation, Boston, MA 02114 (US)
(72) Inventor: PARK, Boris Hyle, Somerville, MA 02143 (US); DE BOER, Johannes F., Somerville, MA 02145 (US)
(74) Representative: Kopf, Korbinian Paul
(86) International application number: PCT/US2007/060670
(87) International publication number: WO 2007/100935

(56) References cited:
- WO-A-2004/088361
- US-A- 6 037 579
- US-A1- 2004 100 631
- SCHMITT J M ET AL: "SPECKLE IN OPTICAL COHERENCE TOMOGRAPHY: AN OVERVIEW" PROCEEDINGS OF THE SPIE, SPIE, BELLINGHAM, VA, US, vol. 3726, 6 October 1998 (1998-10-06), pages 450-461, XP001182620 ISSN: 0277-786X
- YELIN D ET AL: "Double-clad fiber for endoscopy" OPTICS LETTERS, OSA, OPTICAL SOCIETY OF AMERICA, WASHINGTON, DC, US, vol. 29, no. 20, 15 October 2004 (2004-10-15), pages 2408-2410, XP002347219 ISSN: 0146-9592
- JOSEPH M SCHMITT: "Optical Coherence Tomography (OCT): A Review" IEEE JOURNAL OF SELECTED TOPICS IN QUANTUM ELECTRONICS, IEEE SERVICE CENTER, PISCATAWAY, NJ, US, vol. 5, no. 4, August 1999 (1999-08), XP011062609 ISSN: 1077-260X

## Description

### FIELD OF THE INVENTION

The present invention relates to systems, arrangements and methods for optical imaging using a light beam reflected from a sample surface and compared to a reference light beam, wherein the wave front of light returning from the sample is modified in successive measurements such that acquired data can be used to reduce the appearance of speckle. The exemplary systems, arrangements and methods can allow for reduced speckle contrast within a single Optical Coherence tomography image with little to no reduction in lateral and axial resolution.

### BACKGROUND INFORMATION

Optical coherence tomography is an imaging technique that measures the interference between a reference beam of light and a detected beam reflected back from a sample. A detailed system description of traditional time-domain OCT was first described by Huang et al. (see publication labeled by numeral [1] as indicated herein below). Exemplary detailed descriptions for spectral-domain OCT and optical frequency domain interferometry systems and processes are described in, for example, International Patent Application PCT/US2004/029148, filed September 8, 2004, U.S. Patent Application No. 11/266,779, filed November 2, 2005, and U.S. Patent Application No. 10/501,276, filed July 9, 2004. In all these exemplary systems and processes, a series of depth scans can be compiled to create a cross-sectional image of the sample.

Various extensions can provide additional information on tissue properties over the OCT imaging processes and systems alone. For example, polarization sensitive OCT (PS-OCT) can provide additional systems and processes which utilize the sensitivity to light polarization changing properties of the sample (see publications labeled by numerals [2-6] as indicated herein below). A simultaneous detection of interference fringes in two orthogonal polarization channels may allow for a determination of the Stokes parameters of light (see publication labeled by numeral [6] as indicated herein below). A comparison of the Stokes parameters of the incident state to the state obtained by the reflected signals from the sample can yield a depth-resolved map of optical properties such as birefringence (see publication labeled as [6]). In addition, optical Doppler tomography (ODT) systems and processes are capable of depth-resolved imaging of flow (see publications labeled by numerals [7-10] as indicated herein below). The flow sensitivity can be achieved by measuring the shift in carrier frequency of the interference fringe pattern due to backscattering of light from moving particles, or by comparing the phase of the interference fringe pattern from one A-line to the next. Phase-resolved optical Doppler tomography (ODT) systems and processes (see publications labeled by numerals [9, 10] as indicated herein below) can enable depth-resolved imaging of the flow by observing differences in a phase between successive depth scans.

One of the main sources of speckle in OCT is discussed in Schmitt et al. (see publication labeled by numeral [11] as indicated herein below). In this publication, it was provided that the changes that a focused wave incident on the tissue undergoes as it propagates through the tissue to the sample volume, scatters back, and then propagates once again through the tissue back to the lens should be considered. This publication also provides that two main processes influence the spatial coherence of the returning wave: (1) multiple backscattering of the beam inside and outside of the desired sample volume and (2) random delays of the forward-propagating and returning beam caused by multiple forward scattering. Although the first of these is the primary source of speckle in rough-surface imaging, the second must also be considered in coherent imaging systems like OCT, that utilize penetrating waves. The common feature of both processes (as indicated in this publication) is that they alter the shape of the wave front of the returning beam and create localized regions of constructive and destructive interference that appear as speckle in OCT images (referring to publication labeled [12] as indicated herein below).

For example, the wave front of light returning from a particular location in a sample is modified by other parts of the sample. These other portions of the sample, including nearby regions that contribute to multiple backscatter and regions along the optical path of light returning from the particular location, can be thought of as a filter that alters the shape of the wave front of the returning beam. The effect of the filter creates the localized constructive and destructive interference pattern that forms the resulting speckle pattern. Alterations on the order of a fraction of the wavelength of light used for imaging in the sample will result in a different filter, and consequently, also a different speckle pattern.

The standard deviation of a polarized speckle pattern is equal to its mean intensity. (See publication labeled by numeral [13] as indicated herein below). Consequently, speckle can mask the presence of small, thin, or weakly reflecting structures in intensity images. In addition, the accuracy with which phase can be determined improves with higher signal-to-noise ratio ("SNR"). Regions for which the intensity of reflected light has been suppressed by speckle will therefore have a lower SNR and therefore higher uncertainty in phase determination. Since both polarization-sensitive and Doppler OCT measurements rely on phase determination, the accuracy of polarization property and flow determination will suffer in these localized regions of speckle-derived destructive interference. Therefore, the ability to reduce speckle contrast can be of benefit to traditional intensity based OCT as well as to extensions such as PS-OCT and Doppler OCT.

The size of the smallest structures resolvable by OCT imaging is determined by the lateral and axial resolution of the OCT system. The axial resolution is primarily determined by the spectral bandwidth of the source, while the lateral resolution is governed by the optics with which light is focused into the sample. Modulation of the focus on the sample can yield information on the presence of structures smaller than the lateral resolution.

There are several existing solutions to the problem of speckle reduction in OCT. The simplest method is to simply average a number of normally acquired OCT images of the same sample. Besides requiring multitude of images, the main problem with this method is that while each image has its own speckle pattern, these patterns are often very highly correlated. This is the case for rapidly acquired images in succession, and consequently, the reduction in speckle contrast can be difficult to appreciate.

In particular, polarization diversity can also be used to reduce speckle in OCT images by using an unpolarized source beam and measuring the interference between the reference and sample arms of the interferometer in a polarization-sensitive manner. The reasoning behind this technique is that images generated by the two orthogonal polarization states will have their own speckle patterns. However, the two main disadvantages of this method are that the reduction in the SNR of a speckle pattern is limited, and such a technique cannot be used in a PS-OCT system.

In spatial compounding, the absolute magnitudes of signals derived from the sample volume or slightly displaced sample volumes are averaged to form a new signal with reduced speckle noise. The effectiveness of the technique is determined by the number of signals that are averaged together. A number of previous applications of this technique in OCT have been used in the form of angular compounding, where an array of detectors located in the back Fourier plane of the objective lens receives light backscattered from the same sample volume at different angles (see publication labeled by numeral [11] as indicated herein below). These methods are complicated in terms of system implementation and increase the amount of data that should be acquired.

Frequency compounding uses the reduced correlation between speckled images for different optical wavelength ranges. The correlation between resulting speckle patterns for small sub-bands of the full spectrum of the optical source depends on the size and overlap of the sub-bands (see publication labeled by numeral [11] as indicated herein below). However, any reduction in optical bandwidth will likely result in a loss in an axial resolution, making this method possibly unsuitable for use with OCT.

Further, a number of post-processing methods have been used to reduce speckle in OCT images. These techniques include median filtering, homomorphic Weiner filtering, multi-resolution wavelet analysis, and adaptive smoothing (see publication labeled by numeral [11] as indicated herein below). While several of these techniques are effective, they require extensive computation. WO 2004/088361 A2 discloses a high speed method and apparatus for implementing angular compounding for reducing speckle in OCT images.

Accordingly, it may be beneficial to address and/or overcome at least some of the deficiencies described herein above.

### SUMMARY AND EXEMPLARY OBJECTS OF THE PRESENT INVENTION

One of the objectives of the present invention is to overcome certain deficiencies and shortcomings of the conventional systems and methods (including those described herein above), and the invention provides systems, arrangements and methods for optical imaging using a light beam reflected from a sample surface and compared to a reference light beam, wherein the wave front of light returning from the sample is modified in successive measurements such that acquired data can be used to reduce the appearance of speckle as defined in the claims.

There are several exemplary aspects of exemplary embodiments of the present invention that are beneficial for reducing the speckle contrast. For example:
a. Speckle reduction can be achieved within a single image by averaging small numbers of depth scans with different speckle patterns. Implementation of this technique does not always increase the amount of data that needs to be acquired.
b. Implementation into a pre-existing OCT system by the addition of a wave front altering element in the sample arm beam path.
c. Reduction of speckle contrast in an image can be achieved without complicated post-processing.
d. Application to a more complex imaging system, such as an OCT system designed for adaptive optics, or with optics designed for high-resolution.
e. Application to other variants of OCT, such as polarization-sensitive OCT and Doppler OCT.
f. Detection of structures smaller than the focal volume of light on the sample.

Any OCT imaging system in which coherence light interferes would likely have speckle, and can benefit from the exemplary embodiments of the present invention. For example, a device can be added into the sample beam path of a pre-existing OCT system, to rapidly modify the wave front reflected from the sample back into the imaging system. Averaging a small number of measurements obtained using this exemplary device from the same or nearby locations may reduce the appearance of speckle in resulting images. In addition, proper use and analysis of data obtained while using this device will allow for detection of structures smaller than the focal volume.

According to an exemplary aspect of the present invention, systems and methods for generating information associated with at least one portion of a sample can be provided. For example, it is possible to receive from the at least one portion and/or transmit to at least one electro-magnetic radiation to the portion. At least one first wave front of the electro-magnetic radiation received from and/or transmitted to the portion can be provided to generate at least one first transmitted wave front. After the first transmitted wave front is generated, at least one characteristic of at least one second wave front of the electro-magnetic radiation received from and/or transmitted to the portion can be modified to generate at least one second modified wave front which is different from the first transmitted wave front. The above can be performed by at least one first arrangement. Further. the information can be generated based on the first transmitted wave front and the second modified wave front, e.g., using at least one second arrangement.

In another exemplary aspect of the present invention, at least one characteristic of the first wave front can be modified to generate a first modified wave front prior to the modification of the characteristic of the one second wave front. The information can be generated based on the first and second modified wave fronts. Further information can be generated based on the first and second modified wave fronts, and the further information may be associated with a particular volume of the portion which is smaller than a focal volume of the portion that receives the electro-magnetic radiation. The first arrangement includes a micro-deformable mirror.

According to yet another exemplary aspect of the present invention, the information can be averaged to produce resultant data in which a speckle noise is lower than the speckle noise in the information. The first arrangement may be situated in a catheter arrangement. The system can include an interferometric arrangement which may receive at least the first and second wave fronts from a sample arm and a further electro-magnetic radiation from a reference arm. The first and second wave fronts may be associated with approximately the same focal volume within the portion of the sample. The characteristic of the second wave front of the electro-magnetic radiation can be a phase of the second wave front , and wherein the phase can be modified by at most approximately 2.

In a further exemplary aspect of the present invention, it is possible to compensate for a predetermined wave front distortion associated with the portion of the sample and/or an optical beam path within the first arrangement and/or the second arrangement. The first arrangement can further include at least one optical fiber and/or a structure having a pinhole. This arrangement can select at least one portion of the electro-magnetic radiation received from the portion of the sample. The information may be associated with a reflectivity of, a motion within and/or a polarization property of the at least one portion. A further arrangement can be provided which is configured to scan the portion using the electro-magnetic radiation over the portion to generate further information which is associated with a two-dimension image and/or a three-dimensional image of the portion (e.g., an anatomical structure).

Other features and advantages of the present invention will become apparent upon reading the following detailed description of embodiments of the invention, when taken in conjunction with the appended claims.

### BRIEF DESCRIPTION OF THE DRAWINGS

Exemplary objects, features and advantages of the invention will become apparent from the following detailed description taken in conjunction with the accompanying figures showing illustrative embodiments of the invention, in which:
Figure 1 is an illustration of an exemplary embodiment of a reflective method of a wave front modification to effect speckle reduction according to the present invention;
Figure 2 is an illustration of an exemplary embodiment of a transmission method of the wave front modification to effect the speckle reduction according to the present invention;
Figure 3 is a flow diagram of an exemplary embodiment of a process in accordance with the present invention;
Figure 4 is an illustration of an exemplary implementation of a use of the system and process in accordance with an exemplary embodiment of the present invention, which uses a collimator that directs a beam to a micro-deformable mirror;
Figure 5 is an illustration of an exemplary implementation of a use of the system and process in accordance with an exemplary embodiment of the present invention, which uses additional lenses in a telecentric configuration;
Figure 6 is a scatter plot of exemplary lateral and axial resolutions resulting from various micro-deformable mirror patterns generated in accordance with exemplary embodiments of the present invention;
Figure 7 is a graph of exemplary signal to noise ratio for increasing amplitude micro-deformable mirror patterns generated in accordance with exemplary embodiments of the present invention;
Figure 8 are intensity and phase retardation images for an exemplary sample of a chicken muscle imaged with a single stationary pattern (left pair) and alternating between two different mirror patterns (right pair);
Figure 9 are graphs of axial FWHM, intensity speckle contrast ratio, and standard deviation of phase retardation plots for the exemplary sample of the chicken of Figure 8 in accordance with the exemplary embodiments of the present invention; and
Figure 10 are images for a region of an exemplary human fingertip acquired in vivo using a stationary mirror pattern (left set) and alternating between two different mirror patterns (right set) in accordance with the exemplary embodiments of the present invention.

Throughout the figures, the same reference numerals and characters, unless otherwise stated, are used to denote like features, elements, components or portions of the illustrated embodiments. Moreover, while the subject invention will now be described in detail with reference to the figures, it is done so in connection with the illustrative embodiments.

### DETAILED DESCRIPTION OF EXEMPLARY EMBODIMENTS

Exemplary embodiments of the present invention include certain modifications to the sample arm of an exemplary OCT imaging system, and may also include a device to modify the detected spatial mode of light returning from a sample (e.g., modifying the wave front of light returning from the sample). For example, a small number of measurements acquired from the same or nearby locations with different settings of the device can be averaged together in such a way to reduce speckle contrast in a single image with little loss in axial or lateral resolution. This may provide for better imaging of small, thin, or weakly reflecting structures that might otherwise be masked or difficult to resolve due to the presence of speckle. Data obtained while using the device can also be used to detect the presence of structures smaller than the focal volume.

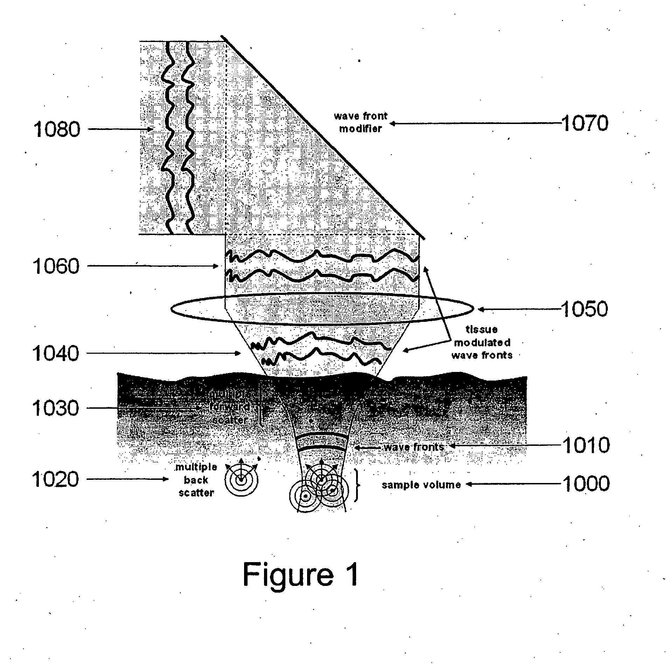

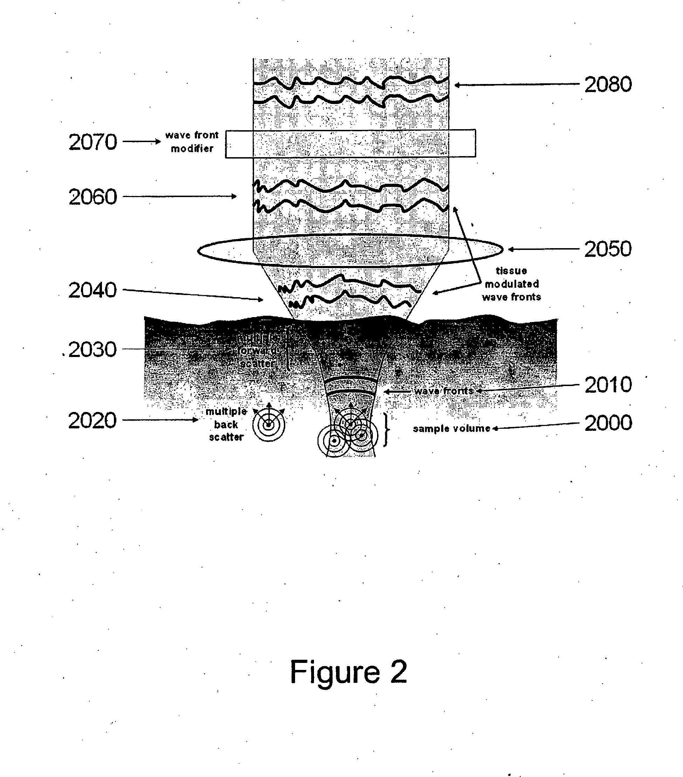

Figure 1 provides an illustration of an exemplary embodiment of a reflective method of a wave front modification to effect speckle reduction according to the present invention. Figure 2 provides an illustration of an exemplary embodiment of a transmission method of the wave front modification to effect the speckle reduction according to the present invention. For example, the wave front of light returning (1010 of Figure 1 and 2010 of Figure 2) from the focal volume (1000 of Figure 1 and 2000 of Figure 2) of a sample can be modulated by other parts of the sample (1020, 1030 of Figure 1, and 2020, 2030 of Figure 2), likely giving rise to a specific speckle issue. An element (1070 and 1080 of Figure 1) can be introduced in the sample optical beam path to further modify the wave front, resulting in a different speckle pattern. The wave front modifications can be varied for successive depth or lateral scans, such that these scans will have different speckle patterns. Averaging small numbers of scans will then result in an image with reduced speckle contrast with no change in the structure of derived images.

Two exemplary ways of introducing such wave front which is capable of modifying optical element in the sample arm beam path may be via reflection and transmission, as shown in Figures 1 and 2, respectively. In these exemplary methods, the overall path lengths of small beamlets within the full sample light beam are modified by a wave front modification component (1070 of Figure 1 and 2070 of Figure 2) slightly with respect to one another, resulting in an altered wave front for the overall beam (1080 and 2080). By changing this beamlet-modification pattern from depth scan to depth scan, different speckle patterns can be created. The amplitude of these relative movements need only be on the order of fractions of the optical wavelength to significantly alter the speckle pattern. The pattern of relative path length alterations can be modified from one depth scan to the next, and the resulting measurements for a small number of depth scans from the same or nearby regions can be averaged to achieve speckle reduction within a single image. While at least two such depth scans with different speckle patterns are preferable, any number of wave front patterns and depth scans can be used to further improve the reduction of speckle.

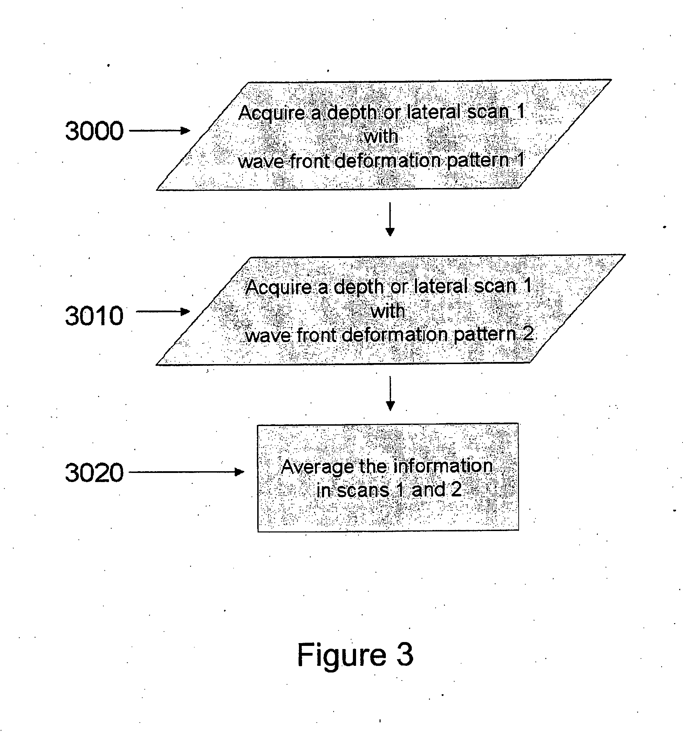

A flow diagram of an exemplary embodiment of the technique in accordance with the present invention is shown in Figure 3. For example, a depth or lateral profile 1 can be acquired with wave front deformation pattern 1 3000, followed by acquisition of profile 2 acquired with wave front deformation pattern 2 3010. The information in depth profiles 1 and 2 can then be averaged to reduce speckle 3020. The amount of speckle reduction can be improved by acquiring and averaging together the information from a greater number of depth profiles corresponding to a greater number of wave front deformation patterns.

In order not to change the axial resolution of the system, it is preferred that the overall beam path length varies by less than the axial resolution of the system from one pattern to the next. Additionally, the lateral resolution of the system can be largely preserved by insuring that there is minimal angular displacement of the sample beam caused by the wave front altering optical element.

The present invention in a reflection geometry includes the use of a micro-deformable mirror (mDM). In various cases, it is only preferable to be able to introduce small relative path length or phase variations between beamlets of the overall sample optical beam such that wave front of the beam is modified, and to be able to vary the pattern of this wave front alteration between successive measurements.

One of the exemplary objects of the present invention is to reduce speckle by averaging small numbers of measurements with different speckle patterns together. By rapidly varying the speckle pattern between acquisition of successive depth scans of an OCT image, a significant speckle reduction can be achieved within a single image. In addition to conventional intensity OCT imaging, the exemplary embodiments of the present invention can be used with extensions or variants of OCT, including, but not limited to the polarization-sensitive OCT and Doppler OCT systems and processes. Since an exemplary application of wave front alteration likely should change the speckle pattern, without the changes to the basic structure of the image, these functional extensions and variants of OCT should not be significantly adversely affected by the exemplary embodiments of the technique(s) in accordance with the present invention. One of the effects of the exemplary embodiments of the present invention on these extensions and variants can be an increase in the minimal detectable phase difference that is resolvable.

The exemplary embodiments of the present invention can also be used in conjunction with an adaptive optics system. Adaptive optics systems generally use a wave front sensor and a wave front altering element to flatten the wave front in the sample beam path in order to improve the lateral resolution of resulting images. The type of wave front modifications preferable for the exemplary embodiments of the present invention can be superimposed on that required for adaptive optics to reduce the appearance of speckle within the resulting images as well.

Another use of the exemplary embodiments of the present invention may be to modify the focus of the beam incident on the sample to detect substructures within the focal volume of the sample. The incident beam focus can be modulated to match small substructures within the focal volume of the sample. For example, the spatial structure of the focus can be designed to give maximum reflection for substructures of a particular spacing and size. A comparison of such a measurement with other measurements with different modulated focal patterns can then yield information on structures smaller than the focal volume in size.

### EXEMPLARY SUPPORTING DATA

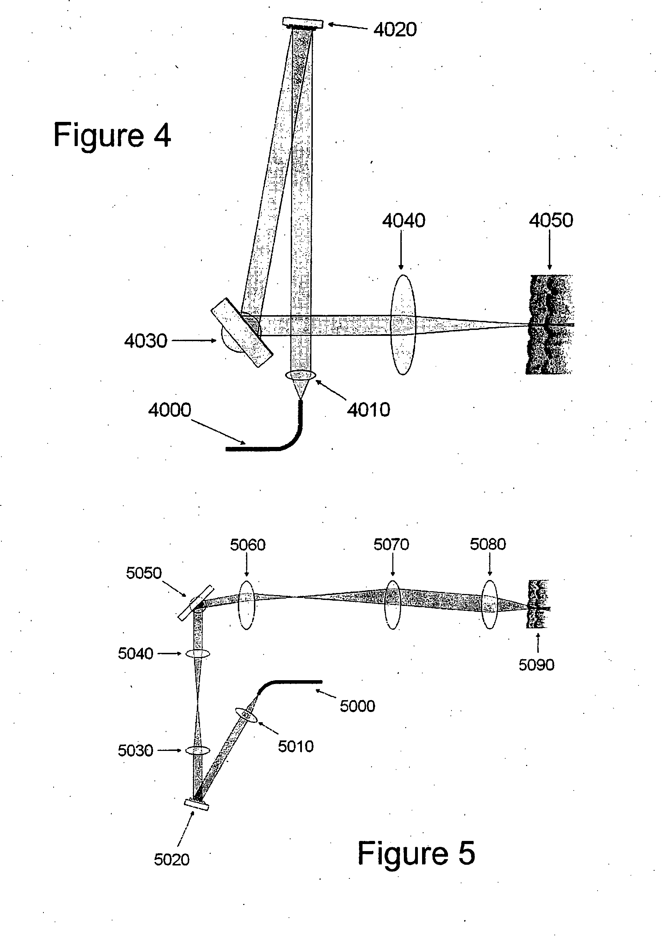

An exemplary implementation of the exemplary embodiment of the present invention using a micro-deformable mirror mDM 4020 is shown in Figure 4. For example, the mDM 4020 has a 3x3 mm square reflective surface which can be modified by 140 actuators in a 12x12 array (the corners of the array are not controllable). The sample arm of a spectral-domain OCT system can be composed of a collimator 4010 that directs a 2 mm diameter beam to the micro-deformable mirror 4020. The light can be reflected on to a galvanometer-mounted mirror 4030, which scans the light on the sample 4050 after it is passed through a focusing lens 4040 (f=60 mm).

Figure 5 is an illustration of an exemplary implementation of a use of the system and process in accordance with an exemplary embodiment of the present invention, which uses additional lenses in a telecentric configuration. For example, the exemplary configuration includes lenses 5010 and 5080 and a collimator 5050 which are similar to the lenses 4010 and 4040 and the collimator 4030, respectively, of Figure 4. In addition, the exemplary configuration of Figure 5 ulilizes a mirror 5020 to direct the beams to the additional lenses 5030 and 5040, which direct the beams to the collimator 5050. The collimator 5040 then directs the beams to the further lenses 5060, 5070, which forward the beams to the sample 5090 through the lens 5080.

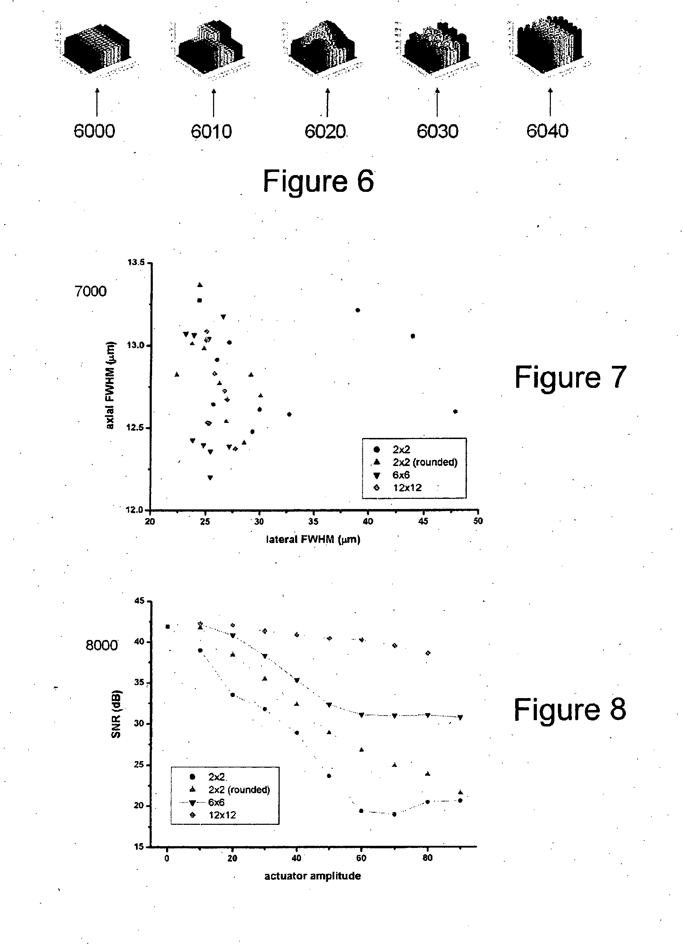

The actuators of the mDM may be set according to certain exemplary patterns with various spatial frequencies and amplitudes such that the overall height of the patterns was constant. The mirror patterns used in this exemplary embodiment can be denoted by 2x2 6000, 2x2s 6010, 6x6 6020, and 12x12 6030, as illustrated in Figure 6. The patterns may be based on checkerboards, where each piece was composed of a number of actuators. For example, for the 12x12 pattern 6030, the height of each actuator alternated between two values centered around the middle height of the actuator. For the 2x2 pattern 6000, the height of squares composed of 36 actuators alternated between values can be calculated in the same manner. The 2x2s pattern 6010 is a sinusoidally-smoothed version of the 2x2 pattern 6000.

The coupling efficiency, lateral and axial resolution of the system in accordance with the exemplary embodiment of the present invention can be determined by imaging a resolution target (e.g., Air Force 1951) for stationary patterns with varying amplitudes. The results of these exemplary measurements 7000 and 8000, respectively, are shown in Figures 7 and 8. For example, the axial FWHM for the different patterns varied from 12.2 to 13.4 microns, which represents an exemplary minimal variation in axial resolution. For various exemplary patterns, the lateral resolution did not change significantly either, ranging from 22 to 31 microns. In certain cases, the lateral resolution degraded, but this degradation can be attributed to reflected angular deviations for the 2x2 pattern. The efficiency of the overall sample imaging system demonstrated a decreasing signal-to-noise ratio (SNR) of the reflected signal for increasing amplitudes of the various patterns. Mirror pattern pairs were chosen based on their SNR values.

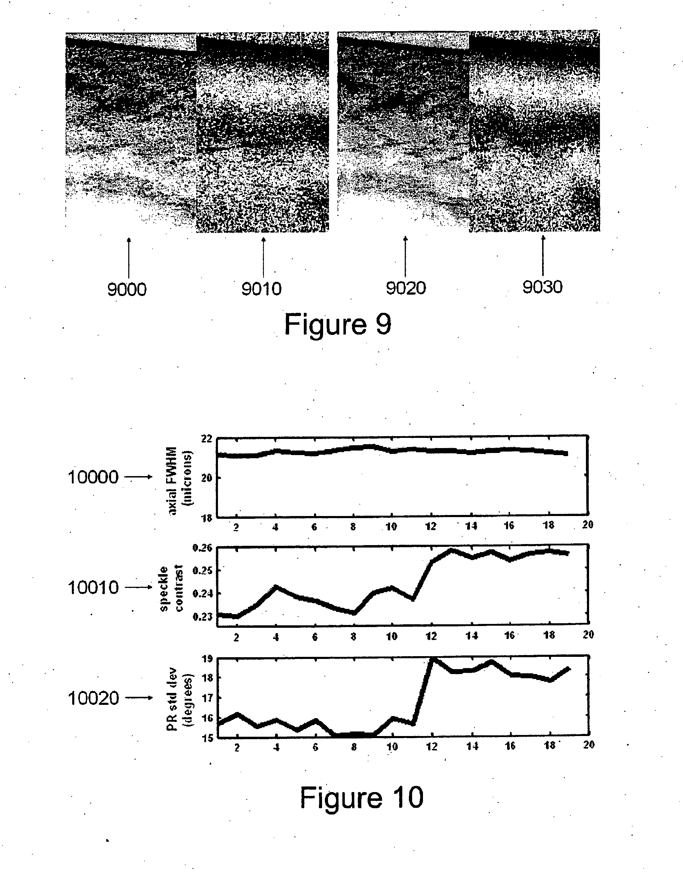

A sequence of images of an exemplary sample of chicken muscle were acquired using the exemplary embodiment of the system and process according to the present invention while alternating the mDM between a 6x6 pattern and a matching 12x 12 pattern at approximately the same rate as the depth scan acquisition rate. The same exemplary sample was then imaged using the stationary 6x6 pattern. The images were composed of 2048x256 pixels covering an area 2x2 mm, and were processed identically by averaging 4 depth scans together. Representative images are shown in Figure 9. The appearance of speckle is visibly reduced for the intensity and polarization images acquired while alternating between mirror patterns 9010, while the overall intensity and phase retardation images remained the same. The exemplary axial resolution as determined from the highly reflective surface of the sample 10000, the exemplary ratio between the standard deviation and mean of the intensity (e.g., speckle contrast ratio) 10010, and the standard deviation of the phase retardation 10020 were determined during the imaging sequence, and shown in Figure 10. The state of the mirror changed from being alternated to stationary between frames 11 and 12. This is indicated by a sharp increase in both the speckle contrast ratio and standard deviation of the calculated phase retardation. There was no significant change in the axial resolution. The data demonstrates that speckle is reduced by alternating the mirror pattern between two states with no loss in resolution.

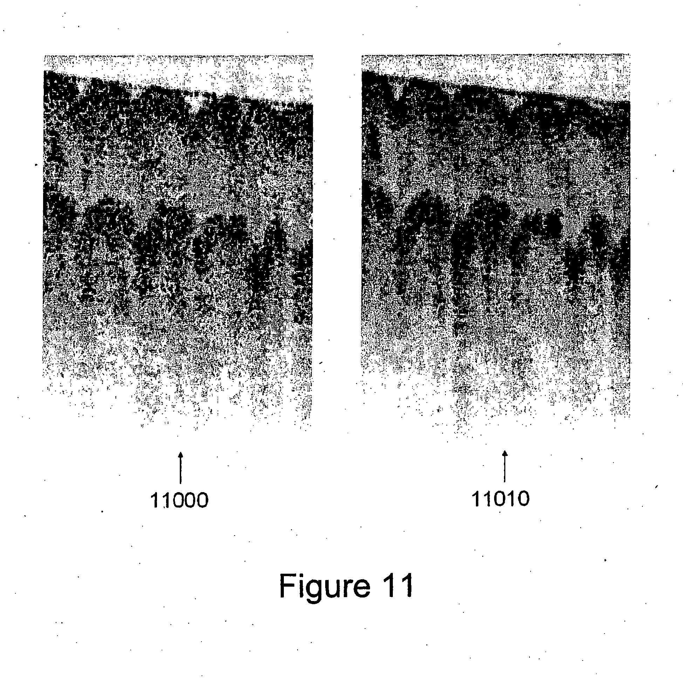

Substantially the same region of a human fingertip was imaged using both a stationary mirror pattern 11000 and alternating between mirror patterns between successive depth scans 11010. The appearance of speckle is illustrated in the images shown in Figure 11. References described herein are as follows
1. Huang, D., Swanson, E.A., Lin, C.P., Schuman, J.S., Stinson, W.G., Chang, W., Hee, M.R., Flotte, T., Gregory, K., Puliafito, C.A., and Fujimoto, J.G., Optical Coherence Tomography. Science, 1991. 254(5035): p. 1178-1181.
2. de Boer, J.F., Milner, T.E., van Gemert, M.J.C., and Nelson, J.S., Two-dimensional birefringence imaging in biological tissue by polarization-sensitive optical coherence tomography. Opt. Lett., 1997. 22(12): p. 934-936.
3. Everett, M.J., Schoenenberger, K., Colston, B.W., and Da Silva, L.B., Birefringence characterization of biological tissue by use of optical coherence tomography. Opt. Lett, 1998. 23(3): p. 228-230.
4. de Boer, J.F., Srinivas, S.M., Malekafzali, A., Chen, Z., and Nelson, J.S., Imaging thermally damaged tissue by polarization sensitive optical coherence tomography. Opt Exp., 1998. 3(6): p. 212-218.
5. Schmitt, J.M. and Xiang, S.H., Cross-polarized backscatter in optical coherence tomography of biological tissue. Opt. Lett., 1998. 23(13): p. 1060-1062.
6. de Boer, J.F., Milner, T.E., and Nelson, J.S., Determination of the depth-resolved Stokes parameters of light backscattered from turbid media by use of polarization-sensitive optical coherence tomography. Optics Letters, 1999. 24(5): p. 300-302.
7. Chen, Z.P., Milner, T.E., Srinivas, S., Wang, X.J., Malekafzali, A., vanGemert, M.J.C., and Nelson, J.S., Noninvasive imaging of in vivo blood flow velocity using optical Doppler tomography. Optics Letters, 1997. 22(14): p. 1119-1121.
8. Izatt, J.A., Kulkami, M.D., Yazdanfar, S., Barton, J.K., and Welch, A.J., In vivo bidirectional color Doppler flow imaging of picoliter blood volumes using optical coherence tomograghy. Optics Letters, 1997. 22(18): p. 1439-1441.
9. Zhao, Y.H., Chen, Z.P., Saxer, C., Xiang, S.H., de Boer, J.F., and Nelson, J.S., Phase-resolved optical coherence tomography and optical Doppler tomography for imaging blood flow in human skin with fast scanning speed and high velocity sensitivity. Optics Letters, 2000. 25(2): p. 114-116.
10. Zhao, Y.H., Chen, Z.P., Saxer, C., Shen, Q.M., Xiang, S.H., de Boer, J.F., and Nelson, J.S., Doppler standard deviation imaging for clinical monitoring of in vivo human skin blood flow. Optics Letters, 2000. 25(18): p. 1358-1360.
11. Schmitt, J.M., Xiang, S.H., and Yung, K.M., Speckle in optical coherence tomography. Journal of Biomedical Optics, 1999. 4(1): p. 95-105.
12. Wax, A. and Thomas, J.E., Measurement of smoothed Wigner phase-space distributions for small-angle scattering in a turbid medium. Journal of the Optical Society of America a-Optics Image Science and Vision, 1998. 15(7): p. 1896-1908.
13. Dainty, J.C., Laser Speckle and Related Phenomena. 1984: Springer-Verlag.

The foregoing merely illustrates the principles of the invention. Various modifications and alterations to the described embodiments will be apparent to those skilled in the art in view of the teachings herein. Indeed, the arrangements, systems and methods according to the exemplary embodiments of the present invention can be used with and/or implement any OCT system, OFDI system, SD-OCT system or other imaging systems, and for example with those described in International Patent Application PCT/US2004/029148, filed September 8, 2004, U.S. Patent Application No. 11/266,779, filed November 2, 2005, and U.S. Patent Application No. 10/501,276, filed July 9, 2004.

## Claims

1. An Optimal Coherence Tomography system (OCT) for generating information which is an averaged image associated with a portion of a sample, comprising:
a source of coherent electromagnetic radiation,
a first arrangement comprising a micro-deformable mirror which is configured to spatially modify a wave front of the electro-magnetic radiation transmitted to the sample,
wherein the first arrangement is configured to:
i) transmit to the portion the electro-magnetic radiation,
ii) provide a first wave front of the electro-magnetic radiation transmitted to the portion to generate a first transmitted wave front,
iii) after the first transmitted wave front is generated, modify by the micro-deformable mirror a characteristic of a second wave front of the electro-magnetic radiation transmitted to the portion
to generate a second modified wave front which is different from the first transmitted wave front, wherein a direction of a transmission of the second modified wave front when entering the sample is opposite to a direction of a transmission of the second modified wave front when exiting the sample; and
a further arrangement which is configured to laterally scan the portion or to carry out a depth scan of the portion to generate a first speckled image associated with the first transmitted wave front and a second speckled image associated with the second transmitted wave front, and
a second arrangement which is configured to generate the averaged image based on the first speckled image associated with the first transmitted wave front and the second speckled image associated with the second modified wave front.

2. An Optical Coherence Tomography system (OCT) for generating information which is an averaged image associated with a portion of a sample, comprising:
a source of coherent electro-magnetic radiation,
a first arrangement comprising a micro-deformable mirror which is configured to spatially modify a wave front of the electro-magnetic radiation received from the sample,
wherein the first arrangement which is configured to:
i) receive from the portion the electro-magnetic radiation,
ii) provide a first wave front of the electro-magnetic radiation received from the portion to generate a first transmitted wave front,
iii) after the first transmitted wave front is generated, modify by the micro-deformable mirror a characteristic of a second wave front of the electro-magnetic radiation received from the portion to generate a second modified wave front which is different from the first transmitted wave front, wherein a direction of a transmission of the second modified wave front when entering the sample is opposite to a direction of a transmission of the second modified wave front when exiting the sample; and
a further arrangement which is configured laterally scan the portion or to carry out a depth scan of the portion to generate a speckled image associated with the first transmitted wave front and a second speckled image associated with the second transmitted wave front, and
a second arrangement which is configured to generate the averaged image based on the first speckled image associated with the first transmitted wave front and the second speckled image associated with the second modified wave front.

3. The system according to claim 1 or 2, wherein the first arrangement modifies at least one characteristic of the first wave front to generate a first modified wave front prior to the modification of characteristic of the second wave front.

4. The system according to claim 3, wherein the second arrangement generates further imaging information based on the first and second modified wave fronts, the further information being associated with a particular volume of the portion which is smaller than a focal volume of the portion that receives the electro-magnetic radiation.

5. The system according to claim I or 2, wherein the first arrangement is situated in a catheter arrangement.

6. The system according to claim 1 or 2, wherein the first and second wave fronts are associated with approximately the same focal volume within the portion of the sample.

7. The system according to claim 1 or 2, wherein the characteristic of the second wave front of the electro-magnetic radiation is a phase of the second wave front, and wherein the first arrangement modifies the phase by at most approximately 2.

8. The system according to claim 1 or 2, wherein the first arrangement further includes an optical arrangement which is configured to compensate for a predetermined wave front distortion associated with the portion of the sample and/or an optical beam path within the first arrangement and/or the second arrangement.

9. The system according to claim 1 or 2, wherein the first arrangement further comprises at least one optical fiber and/or a structure having a pinhole.

10. The system according to claim 1 or 2, wherein the information is associated with a reflectivity of, a motion within and/or a polarization property of the portion.

11. A method for generating information which is an averaged image associated with a portion of a sample, comprising:
a) transmitting to the portion an electro-magnetic radiation by an Optical Coherence Tomography system (OCT) comprising a micro-deformable mirror which is configured to spatially modify a wave front of the electro-magnetic radiation transmitted to the sample,
b) providing a first wave front of the electro-magnetic radiation transmitted to the portion to generate a first transmitted wave front,
c) after the first transmitted wave front is generated, modifying by the micro-deformable mirror a characteristic of a second wave front of the electro-magnetic radiation transmitted to the portion to generate a second modified wave front which is different from the first transmitted wave front, wherein a direction of a transmission of the second modified wave front when entering the sample is opposite to a direction of a transmission of the second modified wave front when exiting the sample; and
d) laterally scanning the portion or carrying out a depth scan of the portion and generating a first speckled image associated with the first transmitted wave front and a second speckled image associated with the second transmitted wave front, and
e) generating the averaged image based on the first speckled image associated with the first transmitted wave front and the second speckled image associated with the second modified wave front.

12. A method for generating information which is an averaged image associated with a portion of a sample, comprising:
a) receiving from the portion an electro-magnetic radiation by an Optical Coherence Tomography system (OCT) comprising a micro-deformable mirror which is configured to spatially modify a wave front of the electro-magnetic radiation received to the sample,
b) providing a first wave front of the electro-magnetic radiation received from the portion to generate a first transmitted wave front,
c) after the first transmitted wave front is generated, modifying by the micro-deformable mirror a characteristic of a second wave front of the electro-magnetic radiation received from the portion to generate a second modified wave front which is different from the first transmitted wave front, wherein a direction of a transmission of the second modified wave front when entering the sample is opposite to a direction of a transmission of the a second modified wave front when exiting the sample; and
d) laterally scanning the portion or carrying out a depth scan of the portion and generating a first speckled image associated with the first transmitted wave front and a second speckled image associated with the second transmitted wave front, and
e) generating the averaged image based on a first speckled image associated with the first transmitted wave front and a second speckled image associated with the second modified wave front.

13. The method according to claim 11 or 12, further comprising:
e) modifying a characteristic of the first wave front to generate a first modified wave front prior to the modification of the characteristic of the second wave front, wherein the information is generated based on the first and second modified wave fronts.

14. The method according to claim 11 or 12, further comprising:
f) compensating with an optical arrangement for a predetermined wave front distortion associated with the at least one portion of the sample and/or an optical beam path within at least one arrangement configured to perform one or more of steps (a)-(d).

15. The method according to claim 11 or 12, further comprising:
g) generating further information based on the first and second modified wave fronts, the further information being associated with a particular volume of the portion which is smaller than a focal volume of the portion that receives the electro-magnetic radiation.

## Patentansprüche

1. Ein optisches Kohärenztomographiesystem (Optical Coherence Tomography System, OCT) zur Gewinnung von Informationen in Form eines gemittelten Bildes im Zusammenhang mit einem Abschnitt einer Probe, aufweisend:
eine Quelle kohärenter elektromagnetischer Strahlung,
eine erste Anordnung, aufweisend einen mikro-verformbaren Spiegel, der so konfiguriert ist, dass er eine Wellenfront der elektromagnetischen Strahlung,
die auf die Probe transmittiert wird, räumlich modifiziert,
wobei die erste Anordnung derart konfiguriert ist, dass
i) die elektromagnetische Strahlung auf den Abschnitt transmittiert wird,
ii) eine erste Wellenfront der elektromagnetischen Strahlung auf den Abschnitt transmittiert wird, um eine erste transmittierte Wellenfront zu erzeugen,
iii) nachdem die erste transmittierte Wellenfront erzeugt wurde, ein Merkmal einer zweiten Wellenfront der elektromagnetischen Strahlung, die auf den Abschnitt transmittiert wird, mittels des mikro-verformbaren Spiegels modifiziert wird, um eine zweite modifizierte Wellenfront zu erzeugen, die sich von der ersten transmittierten Wellenfront unterscheidet, wobei eine Transmissionsrichtung der zweiten modifizierten Wellenfront beim Eintritt in die Probe gegenläufig zu der Transmissionsrichtung der zweiten modifizierten Wellenfront beim Austritt aus der Probe ist; und eine weitere Anordnung, die derart konfiguriert ist, dass der Abschnitt lateral gescannt wird oder ein Tiefenscan des Abschnittes durchgeführt wird, um ein erstes Bild mit Speckle-Effekt zu gewinnen, das mit der ersten transmittierten Wellenfront assoziiert ist, und ein zweites Bild mit Speckle-Effekt zu gewinnen, das mit der zweiten transmittierten Wellenfront assoziiert ist, sowie eine zweite Anordnung, die zur Herstellung des gemittelten Bildes basierend auf dem ersten Bild mit Speckle-Effekt, das mit der ersten transmittierten Wellenfront assoziiert ist, und dem zweiten Bild mit Speckle-Effekt, das mit der zweiten modifizierten Wellenfront assoziiert ist, ausgeführt ist.

2. Ein optisches Kohärenztomographiesystem (OTC) zum Gewinnen von Informationen in Form eines gemittelten Bildes, das mit einem Abschnitt einer Probe assoziiert ist, aufweisend:
eine Quelle kohärenter elektromagnetischer Strahlung,
eine erste Anordnung, aufweisend einen mikro-verformbaren Spiegel, der so konfiguriert ist, dass er eine Wellenfront der elektromagnetischen Strahlung,
die von der Probe empfangen wird, räumlich modifiziert,
wobei die erste Anordnung derart konfiguriert ist, dass
i) sie die elektromagnetische Strahlung von dem Abschnitt empfängt,
ii) sie eine erste Wellenfront der elektromagnetischen Strahlung zur Verfügung stellt, die von dem Abschnitt empfangen wird, um eine erste transmittierte Wellenfront zu erzeugen,
iii) nachdem die erste transmittierte Wellenfront generiert wurde, ein Merkmal einer zweiten Wellenfront der elektromagnetischen Strahlung, die von dem Abschnitt empfangen wird, mittels des mikro-verformbaren Spiegels modifiziert wird, um eine zweite modifizierte Wellenfront zu bilden, die sich von der ersten transmittierten Wellenfront unterscheidet, wobei eine Transmissionsrichtung der zweiten modifizierten Wellenfront beim Eintritt in die Probe der Richtung gegenläufig zu der Transmissionsrichtung der zweiten modifizierten Wellenfront beim Austritt aus der Probe ist; und
eine weitere Anordnung, die derart konfiguriert ist, dass der Abschnitt lateral gescannt wird oder ein Tiefenscan des Abschnittes durchgeführt wird, um ein Bild mit Speckle-Effekt zu gewinnen, das mit der ersten transmittierten Wellenfront assoziiert ist, und ein zweites Bild mit Speckle-Effekt zu gewinnen, das mit der zweiten transmittierten Wellenfront assoziiert ist, sowie
eine zweite Anordnung, die zur Herstellung des gemittelten Bildes basierend auf dem ersten Bild mit Speckle-Effekt, das mit der ersten transmittierten Wellenfront assoziiert ist, und auf dem zweiten Bild mit Speckle-Effekt, das mit der zweiten modifizierten Wellenfront assoziiert ist, ausgeführt ist.

3. System gemäß Anspruch 1 oder 2, wobei die erste Anordnung wenigstens ein Merkmal der ersten Wellenfront modifiziert, um eine erste modifizierte Wellenfront zu erzeugen, bevor das Merkmal der zweiten Wellenfront modifiziert wird.

4. System gemäß Anspruch 3, wobei die zweite Anordnung weitere Abbildungsinformation erzeugt, die auf den ersten und zweiten modifizierten Wellenfronten basiert, wobei die weitere Information mit einem bestimmten Volumen des Abschnittes assoziiert ist, das kleiner als ein Fokalvolumen des Abschnitts, der die elektromagnetische Strahlung empfängt, ist.

5. System gemäß Anspruch 1 oder 2, wobei die erste Anordnung in einer Katheteranordnung positioniert ist.

6. System gemäß Anspruch 1 oder 2, wobei die erste und zweite Wellenfront mit in etwa demselben Fokalvolumen innerhalb des Abschnitts der Probe assoziiert sind.

7. System gemäß Anspruch 1 oder 2, wobei das Merkmal der zweiten Wellenfront der elektromagnetischen Strahlung eine Phase der zweiten Wellenfront ist und wobei die erste Anordnung die Phase um höchstens etwa 2 modifiziert.

8. System gemäß Anspruch 1 oder 2, wobei die erste Anordnung ferner eine optische Anordnung umfasst, die derart konfiguriert ist, dass sie eine vorbestimmte Wellenfrontverzerrung, die mit dem Abschnitt der Probe und/oder einem optischen Strahlengang innerhalb der ersten Anordnung und/oder der zweiten Anordnung assoziiert ist, kompensiert.

9. System gemäß Anspruch 1 oder 2, wobei die erste Anordnung ferner wenigstens einen Lichtleiter und/oder eine Struktur mit einem Nadelloch aufweist.

10. System gemäß Anspruch 1 oder 2, wobei die Informationen mit einem Reflexionsvermögen des Abschnittes, einer Bewegung innerhalb des Abschnittes, und/oder einer Polarisationseigenschaft des Abschnittes assoziiert sind.

11. Verfahren zur Gewinnung von Informationen in Form eines gemittelten Bildes, das mit einem Abschnitt einer Probe assoziiert ist, aufweisend:
a) Transmitieren einer elektromagnetischen Strahlung auf den Abschnitt durch ein optisches Kohärenztomographiesystem (OTC), das einen mikro-verformbaren Spiegel aufweist, der derart konfiguriert ist, dass er eine Wellenfront der elektromagnetischen Strahlung, die auf die Probe transmittiert wird, räumlich modifiziert,
b) Bereitstellung einer ersten Wellenfront der elektromagnetischen Strahlung, die auf den Abschnitt übertragen wird, um eine erste transmittierte Wellenfront zu erzeugen,
c) nachdem die erste transmittierte Wellenfront erzeugt wurde, Modifizieren eines Merkmals einer zweiten Wellenfront der elektromagnetischen Strahlung, die auf den Abschnitt transmittiert wird, mittels des mikro-verformbaren Spiegels, um eine zweite modifizierte Wellenfront zu bilden, die sich von der ersten transmittierten Wellenfront unterscheidet, wobei die Transmissionsrichtung der zweiten modifizierten Wellenfront beim Eintritt in die Probe gegenläufig zur Transmissionsrichtung der zweiten modifizierten Wellenfront beim Austritt aus der Probe ist; und
d) laterales Scannen des Abschnittes oder Ausführen eines Tiefenscans des Abschnittes und Erzeugen eines ersten Bildes mit Speckle-Effekt, das mit der ersten transmittierten Wellenfront assoziiert ist, und eines zweiten Bildes mit Speckle-Effekt, das mit der zweiten transmittierten Wellenfront assoziiert ist, und
e) Erzeugen des gemittelten Bildes auf der Basis des ersten Bildes mit Speckle-Effekt, das mit der ersten transmittierten Wellenfront assoziiert ist, und des zweiten Bildes mit Speckle-Effekt, das mit der zweiten modifizierten Wellenfront assoziiert ist.

12. Verfahren zur Gewinnung von Informationen in Form eines gemittelten Bildes, das mit einem Abschnitt einer Probe assoziiert ist, aufweisend:
a) das Empfangen einer elektromagnetischen Strahlung von dem Abschnitt durch ein optisches Kohärenztomographiesystem (OTC), das einen mikro-verformbaren Spiegel aufweist, der derart konfiguriert ist, dass er eine Wellenfront der elektromagnetischen Strahlung, die auf der Probe empfangen wird, räumlich modifiziert,
b) Bereitstellung einer ersten Wellenfront der elektromagnetischen Strahlung, die von dem Abschnitt empfangen wird, um eine erste transmittierte Wellenfront zu erzeugen,
c) nachdem die erste transmittierte Wellenfront erzeugt wurde, Modifizieren eines Merkmals einer zweiten Wellenfront der elektromagnetischen Strahlung, die von dem Abschnitt empfangen wird, mittels des mikro-verformbaren Spiegels, um eine zweite modifizierte Wellenfront zu bilden, die sich von der ersten transmittierten Wellenfront unterscheidet, wobei die Transmissionsrichtung der zweiten modifizierten Wellenfront beim Eintritt in die Probe gegenläufig zu der Transmissionsrichtung der zweiten modifizierten Wellenfront beim Austritt aus der Probe ist; und
d) laterales Scannen des Abschnittes oder Ausführen eines Tiefenscans des Abschnittes und Gewinnen eines ersten Bildes mit Speckle-Effekt, das mit der ersten transmittierten Wellenfront assoziiert ist, und eines zweiten Bildes mit Speckle-Effekt, das mit der zweiten transmittierten Wellenfront assoziiert ist, und
e) Gewinnen des gemittelten Bildes auf der Basis des ersten Bildes mit Speckle-Effekt, das mit der ersten transmittierten Wellenfront assoziiert ist, und des zweiten Bildes mit Speckle-Effekt, das mit der zweiten modifizierten Wellenfront assoziiert ist.

13. Verfahren gemäß Anspruch 11 oder 12, ferner aufweisend:
Modifizieren eines Merkmals der ersten Wellenfront zur Gewinnung einer ersten modifizierten Wellenfront vor der Modifizierung des Merkmals der zweiten Wellenfront, wobei die Information auf der Basis der ersten und zweiten modifizierten Wellenfronten erzeugt wird.

14. Verfahren gemäß Anspruch 11 oder 12, ferner aufweisend:
Kompensieren einer vorbestimmten Wellenfrontverzerrung mit einer optischen Anordnung im Zusammenhang mit dem wenigstens einen Abschnitt der Probe und/oder einem optischen Strahlengang innerhalb wenigstens einer Anordnung, die derart konfiguriert ist, dass einer oder mehrere der Schritte (a)-(d) durchgeführt werden.

15. Verfahren gemäß Anspruch 11 oder 12, ferner aufweisend:
Gewinnen weiterer Information basierend auf den ersten und zweiten modifizierten Wellenfronten, wobei die weitere Information mit einem bestimmten Volumen des Abschnitts assoziiert sind, das geringer als ein Fokalvolumen des Abschnitts ist, der die elektromagnetische Strahlung empfängt.

## Revendications

1. Système de tomographie par cohérence optique (OCT) pour générer des informations qui sont une image mise en moyenne associée à une partie d'un échantillon, comportant :
une source de rayonnement électromagnétique cohérent,
un premier agencement comportant un miroir micro-déformable qui est configuré pour modifier spatialement un front d'onde de rayonnement électromagnétique transmis à l'échantillon,
dans lequel le premier agencement est configuré pour :
i) transmettre à la partie le rayonnement électromagnétique,
ii) fournir un premier front d'onde du rayonnement électromagnétique transmis à la partie pour générer un premier front d'onde transmis,
iii) une fois que le premier front d'onde transmis est généré, modifier par l'intermédiaire du miroir micro-déformable une caractéristique d'un second front d'onde du rayonnement électromagnétique transmis à la partie pour générer un second front d'onde modifié qui est différent du premier front d'onde transmis, dans lequel une direction d'une transmission du second front d'onde modifié lorsqu'il pénètre dans l'échantillon est opposée à une direction de transmission du second front d'onde modifié lorsqu'il quitte l'échantillon, et
un agencement supplémentaire qui est configuré pour balayer latéralement la partie ou pour effectuer un balayage en profondeur de la partie afin de générer une première image tachetée associée au premier front d'onde transmis et une seconde image tachetée associée au second front d'onde transmis, et
un second agencement qui est configuré pour générer l'image mise en moyenne sur la base de la première image tachetée associée au premier front d'onde transmis et la seconde image tachetée associée au second front d'onde modifié.

2. Système de tomographie par cohérence optique (OCT) pour générer des informations qui sont une image mise en moyenne associée à une partie d'un échantillon, comportant :
une source de rayonnement électromagnétique cohérent,
un premier agencement comportant un miroir micro-déformable qui est configuré pour modifier spatialement un front d'onde de rayonnement électromagnétique reçu en provenance de l'échantillon,
dans lequel le premier agencement est configuré pour :
i) recevoir en provenance de la partie le rayonnement électromagnétique,
ii) fournir un premier front d'onde du rayonnement électromagnétique reçu en provenance de la partie pour générer un premier front d'onde transmis,
iii) une fois que le premier front d'onde transmis est généré, modifier par l'intermédiaire du miroir micro-déformable une caractéristique d'un second front d'onde du rayonnement électromagnétique reçu en provenance de la partie pour générer un second front d'onde modifié qui est différent du premier front d'onde transmis, dans lequel une direction d'une transmission du second front d'onde modifié lorsqu'il pénètre dans l'échantillon est opposée à une direction de transmission du second front d'onde modifié lorsqu'il quitte l'échantillon, et
un agencement supplémentaire qui est configuré pour balayer latéralement la partie ou pour effectuer un balayage en profondeur de la partie afin de générer une première image tachetée associée au premier front d'onde transmis et une seconde image tachetée associée au second front d'onde transmis, et
un second agencement qui est configuré pour générer l'image mise en moyenne sur la base de la première image tachetée associée au premier front d'onde transmis et la seconde image tachetée associée au second front d'onde modifié.

3. Système selon la revendication 1 ou 2, dans lequel le premier agencement modifie au moins une caractéristique du premier front d'onde pour générer un premier front d'onde modifié avant la modification de caractéristique du second front d'onde.

4. Système selon la revendication 3, dans lequel le second agencement génère des informations d'imagerie supplémentaires sur la base des premier et second fronts d'onde modifiés, les informations supplémentaires étant associées à un volume particulier de la partie qui est inférieur à un volume focal de la partie qui reçoit le rayonnement électromagnétique.

5. Système selon la revendication 1 ou 2, dans lequel le premier agencement est positionné dans un agencement de cathéter.

6. Système selon la revendication 1 ou 2, dans lequel les premier et second fronts d'onde sont associés approximativement au même volume focal dans la partie de l'échantillon.

7. Système selon la revendication 1 ou 2, dans lequel la caractéristique du second front d'onde du rayonnement électromagnétique est une phase du seconds front d'onde, et dans lequel le premier agencement modifie la phase au maximum approximativement par 2.

8. Système selon la revendication 1 ou 2, dans lequel le premier agencement comprend en outre un agencement optique qui est configuré pour compenser une distorsion de front d'onde prédéterminée avec la partie de l'échantillon et/ou un trajet de faisceau optique dans le premier agencement et/ou le second agencement.

9. Système selon la revendication 1 ou 2, dans lequel le premier agencement comprend en outre au moins une fibre optique et/ou une structure ayant unun trou d'épingle.

10. Système selon la revendication 1 ou 2, dans lequel les informations sont associées à une réflectivité d'un mouvement dans la partie et/ou d'une propriété de polarisation de la partie.

11. Procédé pour générer des informations qui sont une image mise en moyenne associée à une partie d'un échantillon, comprenant :
a) de transmettre à la partie un rayonnement électromagnétique par l'intermédiaire d'un système de tomographie par cohérence optique (OCT) comportant un miroir micro-déformable qui est configuré pour modifier spatialement un front d'onde du rayonnement électromagnétique transmis à l'échantillon,
b) de fournir un premier front d'onde du rayonnement électromagnétique transmis à la partie pour générer un premiers front d'onde transmis,
c) une fois que le premier front d'onde est généré, de modifier par l'intermédiaire du miroir micro-déformable une caractéristique d'un seconds front d'onde du rayonnement électromagnétique transmis à la partie pour générer un second front d'onde modifié qui est différent du premier front d'onde transmis, dans lequel une direction de transmission du second front d'onde modifié lorsqu'il pénètre dans l'échantillon est opposé à une direction d'une transmission du second front d'onde modifié lorsqu'il quitte l'échantillon, et
d) de balayer latéralement la partie et/ou d'exécuter un balayage en profondeur de la partie et de générer une première image tachetée associée au premier front d'onde transmis et une seconde image tachetée associée au second front d'onde transmis, et
e) de générer l'image mise en moyenne sur la base de la première image tachetée associée au premier front d'onde et de la seconde image tachetée associée au second front d'onde modifié.

12. Procédé pour générer des informations qui sont une image mise en moyenne associée à une partie d'un échantillon, comprenant :
a) de recevoir en provenance de la partie un rayonnement électromagnétique par l'intermédiaire d'un système de tomographie par cohérence optique (OCT) comportant un miroir micro-déformable qui est configuré pour modifier spatialement un front d'onde du rayonnement électromagnétique reçu dans l'échantillon,
b) de fournir un premier front d'onde du rayonnement électromagnétique reçu en provenance de la partie pour générer un premier front d'onde transmis,
c) une fois que le premier front d'onde est généré, de modifier par l'intermédiaire du miroir micro-déformable une caractéristique d'un second front d'onde du rayonnement électromagnétique reçu en provenance de la partie pour générer un second front d'onde modifié qui est différent du premier front d'onde transmis, dans lequel une direction de transmission du second front d'onde modifié lorsqu'il pénètre dans l'échantillon est opposé à une direction d'une transmission du second front d'onde modifié lorsqu'il quitte l'échantillon, et
d) de balayer latéralement la partie et/ou d'exécuter un balayage en profondeur de la partie et de générer une première image tachetée associée au premier front d'onde transmis et une seconde image tachetée associée au second front d'onde transmis, et
e) de générer l'image mise en moyenne sur la base d'une première image tachetée associée au premier front d'onde et d'une seconde image tachetée associée au second front d'onde modifié.

13. Procédé selon la revendication 11 ou 12, comprenant en outre :
e) de modifier une caractéristique du premier front d'onde pour générer un premier front d'onde modifié avant la modification de la caractéristique du second front d'onde, dans lequel les informations sont générées sur la base des premier et second fronts d'onde modifiés.

14. Procédé selon la revendication 11 ou 12, comprenant en outre :
f) de compenser à l'aide d'un agencement optique une distorsion de front d'onde prédéterminée associée à au moins une partie d'échantillon et/ou un trajet de faisceau optique à l'intérieur d'au moins un agencement configuré pour exécuter une ou plusieurs des étapes (a)-(d).

15. Procédé selon la revendication 11 ou 12, comprenant en outre :
g) de générer des informations supplémentaires sur la base des premier et second fronts d'onde modifiés, les informations supplémentaires étant associées à un volume particulier de la partie qui est inférieur à un volume focal de la partie qui reçoit le rayonnement électromagnétique.
